# EUROPEAN PATENT SPECIFICATION

(11) **EP 2 125 023 B1**
(45) Date of publication and mention of the grant of the patent: **16.10.2013**
(21) Application number: 08720210.7
(22) Date of filing: 25.01.2008
(51) Int. Cl.: A61K 47/48, A61K 31/167, A61K 31/19, A61P 33/02, A61P 25/00, A61P 25/28, A61P 35/00, A61P 25/08, A61P 3/10

(54) **SOLUBLE FORMS OF INCLUSION COMPLEXES OF HISTONE DEACETYLASE INHIBITORS AND CYCLODEXTRINS, THEIR PREPARATION PROCESSES AND USES IN THE PHARMACEUTICAL FIELD**
LÖSLICHE FORMEN VON EINSCHLUSSKOMPLEXEN AUS HISTONDEACETYLASE-HEMMERN UND CYCLODEXTRINEN, IHRE HERSTELLUNGSVERFAHREN UND VERWENDUNGEN IM PHARMAZEUTISCHEN BEREICH
FORMES SOLUBLES DE COMPLEXES D'INCLUSION D'INHIBITEURS DE L'HISTONE DÉACÉTYLASE ET DES CYCLODEXTRINES, PROCÉDÉS DE PRÉPARATION DE CELLES-CI, ET UTILISATIONS DE CELLES-CI DANS LE DOMAINE PHARMACEUTIQUE

(30) Priority: 26.01.2007 IT RM20070038
(43) Date of publication of application: 02.12.2009
(73) Proprietor: Universita'Degli Studi di Roma "La Sapienza", 00185 Roma RM (IT)
(72) Inventor: DI RIENZO, Barbara, I-00185 Roma RM (IT); MAI, Antonello, I-00185 Roma RM (IT)
(74) Representative: Raimondi, Adriana
(86) International application number: PCT/IT2008/000040
(87) International publication number: WO 2008/090585

(56) References cited:
- EP-A- 0 226 304
- EP-A- 0 268 215
- WO-A-2006/120456
- WO-A-2008/002862
- US-A1- 2005 227 915
- DATABASE CA [Online] CHEMICAL ABSTRACTS SERVICE, COLUMBUS, OHIO, US; 2 August 2007 (2007-08-02), OKAMOTO, AKIO: "Trichostatin A-containing pharmaceuticals for treatment of eye diseases" XP002496504 retrieved from STN Database accession no. 2007:838020 & JP 2007 191398 A (SAISENTAN IGAKU KENKYUSHO K. K., JAPAN) 2 August 2007 (2007-08-02)
- DATABASE WPI Week 198148 Thomson Scientific, London, GB; AN 1981-88173D XP002496505 & JP 56 133236 A (KYOWA HAKKO KOGYO KK) 19 October 1981 (1981-10-19) -& JP 56 133236 A (KYOWA HAKKO KOGYO KK) 19 October 1981 (1981-10-19)
- HOCKLY EMMA ET AL: "Suberoylanilide hydroxamic acid, a histone deacetylase inhibitor, ameliorates motor deficits in a mouse model of Huntington's disease" PROCEEDINGS OF THE NATIONAL ACADEMY OF SCIENCES OF USA, NATIONAL ACADEMY OF SCIENCE, WASHINGTON, DC.; US, vol. 100, no. 4, 18 February 2003 (2003-02-18), pages 2041-2046, XP002389453 ISSN: 0027-8424
- PAULI, WAYNE A. ET AL: "Interaction of pharmaceuticals with Schardinger dextrins. V. Interaction with a series of phenyl-substituted carboxylic acids" JOURNAL OF PHARMACEUTICAL SCIENCES , 54(12), 1745-50 CODEN: JPMSAE; ISSN: 0022-3549, 1965, XP002496502
- REKHARSKY M ET AL: "1:1 and 1:2 Complexation thermodynamics of [gamma]-cyclodextrin with N-carbobenzyloxy aromatic amino acids and [omega]-phenylalkanoic acids" JOURNAL OF THE AMERICAN CHEMICAL SOCIETY 20001108 US, vol. 122, no. 44, 8 November 2000 (2000-11-08), pages 10949-10955, XP002496503 ISSN: 0002-7863
- MANFRED JUNG ET AL: "Amide Analogues of Trichostatin A as Inhibitors of Histone Deacetylase and Inducers of Terminal Cell Differentiation" JOURNAL OF MEDICINAL CHEMISTRY, US AMERICAN CHEMICAL SOCIETY. WASHINGTON, vol. 42, no. 22, 4 November 1999 (1999-11-04), pages 4669-4679, XP002144226 ISSN: 0022-2623

## Description

### Field of the invention

The present invention relates to the preparation of soluble forms of inclusion complexes of the histone deacetylase inhibitor SAHA and cyclodextrins, their preparation and related uses. The preparation process is based on the use of microwaves.

In the description here below the histone deacetylase inhibitors are also referrred to as HDAC inhibitors or simply as HDACIs and the cyclodextrins as CDs.

### Background art

Histones are small basic proteins that interact with the DNA, organizing it or, better, compacting it in such a way as to allow the cells to store it in a restricted volume such as that of the nucleus. Histones are proteins consisting of a globular domain and a flexible amine terminal, the histone tail, which projects from the nucleosome.

The histones (described by Monneret C. European J. Med. Chem. 40 (2005) 1-13), once regarded as static structural elements, are known today as integrating and dynamic components of the regulation of gene expression and of other processes such as chromosomal replication, repair, recombination and segregation. In fact, a whole series of post-translation changes take place on the histone tails: acetylation, phosphorylation, methylation, ubiquitination and ADP-riboxylation.

The most studied of the histone post-translation changes is acetylation. The acetylation state of the histone core is controlled by the competitive activities of the HAT (histone acetyltransferase) and HDAC (histone deacetylase) enzyme superfamilies.

HDAC brings about the deacetylation of lysine residues at the level of the terminal amine tails of the histone proteins, thus giving rise to the compacting of the chomatin structure (heterochromatin) and inhibiting transcription of the genome. Acetylation of the histones, catalyzed by the enzyme HAT, on the other hand, induces a dynamic transition towards a conformationally open chromatin state (euchromatin) which allows gene transcription and thus cell differentiation. This dynamic equilibrium in the acetylation level is therefore an important regulator of gene expression, and a dysfunction of this equilibrium has been shown to be a key event in the genesis and progression of tumours.

Recent studies have demonstrated that HDAC inhibitors are capable of inducing arrest of the cell cycle, differentiation and/or apoptosis and for this reason are nowadays considered to be a promising class of new generation anticancer agents.

In the past ten years numerous HDAC inhibitors have been reported (Miller T.A. et al. J. Medicinal Chem. (2003) Vol. 46, No. 24 5097-5116) as a useful means for studying the acetylation and deacetylation function of chromatin and gene expression. We may distinguish between natural and synthetic compounds.

Among the compounds obtained synthetically, are the derivatives of short and aromatic chain fatty acids, such as, for example, 4-phenylbutyrate and valproic acid, and the derivatives of hydroxamic acid, such as, for example, SAHA (suberoylanilide hydroxamic acid), its aza-analogue pyroxamide and related hybrid second generation compounds (HPCs), the straight chain analogues of TSA (trichostatin) and SAHA, 1,4-cyclohexylene and 1,4-phenylene-N-hydroxycarboxamide, scriptaid, oxamflatin and related compounds, the cyclic hydroxamic acid containing peptides (CHAPs), NVP-LAQ824 and related compounds, benzamide derivatives with MS-275 as the lead compound, trifluoromethyl and other electrophilic ketones (α-ketoamides and heterocyclic ketones). These and other HDACIs are indicated here below with the respective structural formulae.

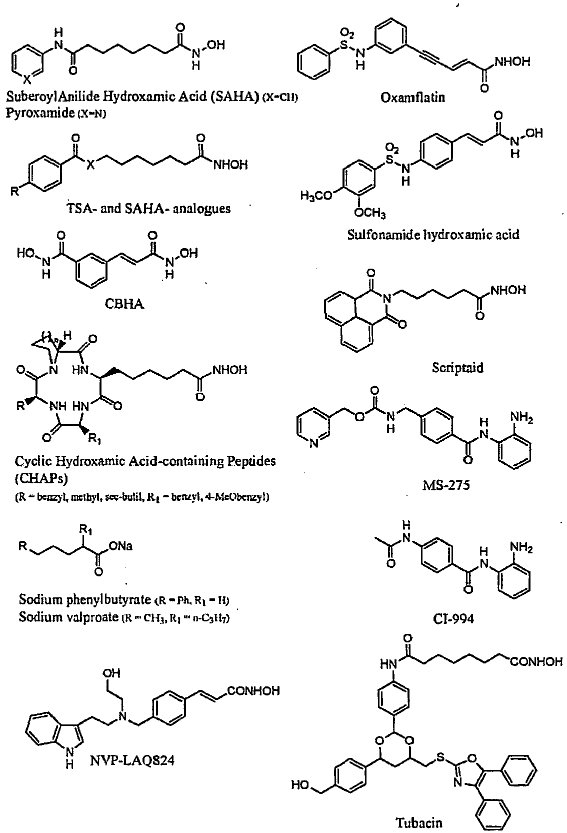

The hydroxamic acids were among the first compounds to be identified as histone deacetylase inhibitors, helping to define the pharmacophoric model of this enzyme.

SAHA (synthesizable as described by Mai A. et al. Oppi Briefs, (2001) Vol. 33, No. 4, 391-394) is the prototype of a series of synthetic hydroxamic acids, the HPCs, with inhibitory activity against HDAC at nanomolar concentrations. Currently in phase II development, it has been shown to be active, at non-toxic doses, in patients with solid tumours and with Hodgkin's lymphoma. Structure-activity studies on HPC derivatives have shown that the hydroxamic portion is fundamental for the inhibitory activity: substitution or modification of this group reduces or annuls the antiHDAC effect.

Hyperacetylation of histones has been determined by means of tumour biopsies after administration of SAHA at doses lower than the effective doses. The first clinical trials have yielded positive results both in patients with solid tumours and in those with tumours of the blood.

Hydroxamates analagous to SAHA are CBHA, pyroxamide and 3-Cl-UCHA, all effective as HDAC inhibitors and as antiproliferation agents both in vitro and in vivo.

TSA, SAHA and CBHA have been the pilot compounds in the design of new HDAC inhibitors with a hydroxyamic function.

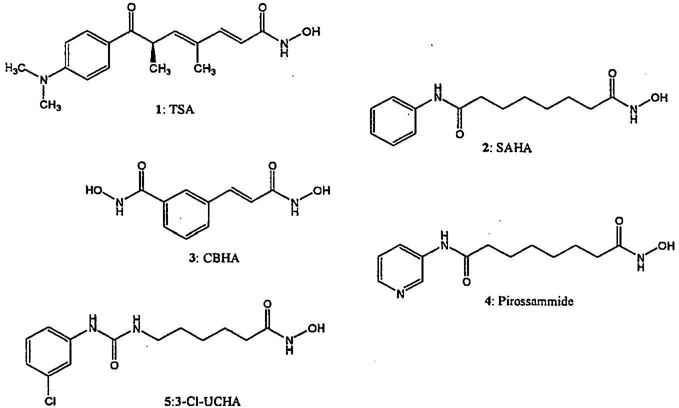

The members of these classes are divided into two groups on the basis of the structure of their linkers:
- inhibitors with linear linkers (TSA and SAHA derivatives)
- inhibitors containing a cyclic or heterocyclic ring (CBHA derivatives).

Belonging to the former group are oxamflatin, scriptaid and a number of SAHA analogues containing phenylalanine; belonging to the latter group, with an aromatic ring as the linker, the first was CBHA, which was then followed by a series of analogous compounds, including NVP-LAQ824, currently in clinical trial phase II.

Another important class of inhibitors with an α,β-unsaturated hydroxamic function comprises the aroyl-pyrrolyl-hydroxyamides (APHAs), in which a pyrrol ring is substituted for the benzene ring (Mai A. et al., J. Med. Chem. 2003, 46, 512-524).

The potential anticancer activity of HDAC inhibitors derives from their ability to influence many cell processes which are impaired in neoplastic cells. Activation of differentiation programs, inhibition of the cell cycle and induction of apoptosis are the main anticancer activities of these compounds. In addition, activation of the host immune response and inhibition of angiogenesis may be important in the *in-vivo* tumour regression mediated by HDAC inhibitors.

Recent studies have highlighted the importance of HDAC inhibitors in fields other than oncology and thus the possibility of finding new cures for severe diseases, such as, for example, for the treatment of the haematic phase of *P. falciparum* and for the tripanosomiases caused by leishmania and tryposoma. (Mai A. et al., Antimicrobial Agents and Chemotherapy, Apr. 2004, 1435-1436).

Recent studies also involve the use of HDAC in the phenomena related to ageing (a senescence-specific form di HDAC has been identified), mental retardation and neurodegeneration.

Nicotinamide, another class II deacetylase inhibitor, is often used in anxiety, osteoarthritis, psychoses, and in the clinical phase of studies for the treatment of a number of types of cancer and for type I diabetes. Lastly, valproic acid, a drug commonly used for epilepsy but with HDAC inhibitory activity, has been studied for potential use in the therapy of spinal muscular atrophy.

On the basis of what has been said, the importance of such drugs emerges and the fundamental role they may play in the treatment of these serious diseases; furthermore, they appear to present a certain selectivity of action, a characteristic which has earned them the attribute of "intelligent drugs", despite the fact that the mechanisms responsible for this phenomenon are not entirely clear.

Most of these drugs, however, have poor affinity for water, and, in particular, poor wettability, poor solubility and a very low dissolution rate. In fact, most of the tests in vitro and also *in vivo* are conducted by solubilizing the compounds in dimethyl-sulphoxide (DMSO) which is known to be a toxic solvent.

Another major problem with these drugs is related to the side effects of oral administration; they are, in fact, poorly tolerated and may indeed cause gastrointestinal haemorrhage, with the result that they must necessarily be taken with food, preferably fatty foods (see: Side effects of the Merck product Zolinza®). In any event, their poor or lion-existing solubility in water makes them poorly bioavailable. It is known, in fact, that one characteristic essential for bioavailability is having a certain degree of solubility in water, since biological liquids are by their very nature aqueous. From a practical point of view, it is, in fact, indispensable that the drugs should possess the correct ratio of hydrophilia to lipophilia, a ratio which is particularly important when, through chemical and/or physical manipulations, one wishes to enhance the therapeutic efficacy of a compound.

Moreover, the fact that a drug can be dissolved in water enables it to be administered orally, which is of by no means negligible importance; in fact, this latter ability presents considerable advantages which cannot be underestimated in the design phase of a drug, particularly with regard to the compliance factor. The willingness of a patient to accept a therapy is of decisive importance for the proper implementation of that therapy. The relative easiness with which a given form of chemotherapy can be complied with when the drug is administered orally is part of a new way of conceiving chemotherapy, better defined as "chemotherapy at home" (Bottomley A, The cancer patient and quality of life. The Oncologist 2002,7; 120-5).

To solve these problems it is therefore very important to use methods for increasing the solubility of HDACIs in water, that is to say methods which are relatively simple, rapid and possibly inexpensive, or, in other words, methods that can be readily transferred to the industrial setting.

It is known that the solubility of a molecule in water can be increased by acting upon its physicochemical characteristics, such as its crystalline or amorphous structure or the size of its particles, or upon its chemical characteristics, e.g. its solubility can be increased, where possible naturally, by transforming it into a soluble salt. A micronization process can be used in such a way as to increase the surface area and thus the surface in contact with the solvent.

An easy method of increasing the solubility and the dissolution rate of a compond, allthough not always feasible for reasons that will be explained here below, consists in the formation of complexes with cyclodextrins.

Cyclodextrins are cyclic oligosaccharides consisting of glucopyranose units linked by means of alpha-1,4 bonds; on the basis of the number of units they are classified as alpha-cyclodextrins, comprising six monosaccaride units, beta-cyclodextrins comprising seven units and gammacyclodextrins comprising eight units. These are obtained by hydrolysis and enzyme cycling of the starch. Many other semisynthetic cyclodextrins have also been produced.

Cyclodextrins possess the ability to form solid inclusion complexes (so-called host-guest complexes) with many substances by means of molecular complexation mechanisms (an example with SAHA is described by Hockey E. et al., PNAS 2003, Vol. 100, No. 4, 2041-2046). In such complexes the guest molecules arrange themselves within the cyclodextrin lipophilic cavity: this cavity, in fact, produces a micromilieu in which non-polar molecules of suitable size can be received without breakage or the formation of covalent bonds. In solution the host-guest system is fixed but is the result of a dynamic equilibrium and depends on the ability of the atoms of the guest molecule to interact locally with those of the cavity; the best solvent for the formation of such complexes is water.

The patent application US2005/227915 relates to a method for treating a series of neurological disorders arising from polyglutamine expansion based on administering to patients an effective amount of one or more acetylase inhibitors. In particular, the acetylase inhibitor considered is SAHA, which is used as a complex with β-cyclodextrin. Preparation of said complex with β-cyclodextrin is carried out as described in the application, page 6, paragraph [0077], i.e., by dissolving the cyclodextrin in water and subsequently adding the SAHA while heating until dissolved.

Hockly E. et al., Proceedings of the National Academy of Science of USA. National Academy of Science. Washington, DC. vol. 100, n° 4, 18 February 2003 (2041-2046) discloses the same experiments and results as those described in US2005/227915. No mention is explicitly made in Hockly E. et al. and US2005/227915 with regards to the heating temperature and duration of the complexes. Moreoyer no mention whatsoever is made in both documents to the use of microwaves during the preparation process of the complex.

Inclusion in cyclodextrins profoundly modifies the physicochemical characteristics of the guest; an increase in solubility in water occurs, in fact, even for highly hydrophobic molecules, as well as the stabilization of molecules showing instability in relation to oxidation, radiation and acidic or basic milieus.

The ability of cyclodextrins to form inclusion complexes depends on two main factors: the first is a steric factor, in that, if the size of the guest molecule is not appropriate, it will be received with difficulty in the lipophilic cavity; the second factor has to do with the thermodynamics of the interaction between the different components of the system (cyclodextrin, guest molecule and solvent), in that, for the complex to form, there must be net energy favourable to the entry of the guest into the cyclodextrin.

Whereas the initial equilibrium, aimed at the formation of the complex, sets in within only a few minutes, it may take much longer to reach the final equilibrium In fact, within the cavity, the guest molecule effects a number of conformational adaptations for the purposes of deriving the maximum advantage from the weak Van der Waals forces present. These interactions come into play to modulate the bioavailability of the drug included in the cyclodextrins.

Beta-cyclodextrin is the most common in nature and the one most often used; it possesses twenty-one hydroxyl groups, seven of which primary and fourteen secondary, and because of its characteristics is suitable for complexing above all molecules of an aromatic nature, unlike alphacyclodextrin which prefers low-molecular-weight molecules with aliphatic chains, and gamma-cyclodextrin, capable of receiving largersized molecules such as macrocycles and steroids.

These different characteristics depend on the different number of glucoside units, which determines the internal diameter and the volume of the cavity.

Cyclodextrins, both natural and modified, are thus used to modify a number of properties of drugs such as solubility, dissolution rate, stability and bioavailability.

Generally, the maximum increase in absorption is obtained when the minimum amount of cyclodextrin necessary for the solubilization of the drug is used; in fact, a substantial excess of cyclodextrin reduces the free fraction of the drug and its consequent availability.

One particular class of semisynthetic cyclodextrins marketed by Roquette consists of: HP-beta-CD and M-beta-CD. The former, marketed under the name of Kleptose HP®, comes in the form of an amorphous white powder, and has an average molecular weight of 1399 daltons, and solubility in water (g/100 ml) of 65% at 25°C and 80% at 50°C; it has a maximum moisture content of 5% and possesses good stability in both an acidic and basic milieu; it can be used for the administration of drugs both by injection and by the oral route.

M-beta-CD, marketed under the name of Kleptose Crysmeb®, is a mixture of cyclodextrins containing from one to seven methyl groups. Kleptose Crysmeb® exp has an average of 4 methyls per molecule of cyclodextrin; it has an average molecular weight of 1191 daltons, and comes as white powder which is easily dissolved in water; its solubility increases with temperature and it is very stable in both an acidic and basic milieu.

WO03/053475 suggests that the use of microwaves may be a rapid method for the preparation of inclusion complexes in cyclodextrins with non-steroidal pain-killers, or with calcium antagonists, or with serum-glucose-lowering agents. The process described is based on the preparation of an aqueous-based pasty mixture of the drug and cyclodextrin; use is also made of additional solvents such as alcohol, acids or bases to favour the processes of dissolution in the very small amount of water added. Microwaves make it possible to obtain inclusion complexes very rapidly, a factor which is by no means negligible if the intention is to transfer the process to an industrial setting, but the dissolution of the drug does not always prove optimal and further filtration and purification steps may be necessary to eliminate the residues of solvents added to the water. Furthermore, the microwaves are applied at high power and this may lead to decomposition of the molecules.

### Summary of the invention

It has now been found and it is an object of the present invention, a class of inclusion complexes of the histone deacetylase inhibitor SAHA, in cyclodextrins which, when solubilized with the aid of microwaves, shows increased activity and bioavailability.

Another object of the invention is the process for the formation of said inclusion complexes, based on the preparation of an aqueous dispersion of the drug in an aqueous solution containing cyclodextrin, said dispersion being transformed into a solution by means of the application of microwaves.

Other objects of the invention will be evident from the detailed descrtion of the invention.

### Brief description of the Figure

Figure 1 illustrates the acetylation kineics on mouse spleen after *in-vivo* administration of the SAHA/CD complex.

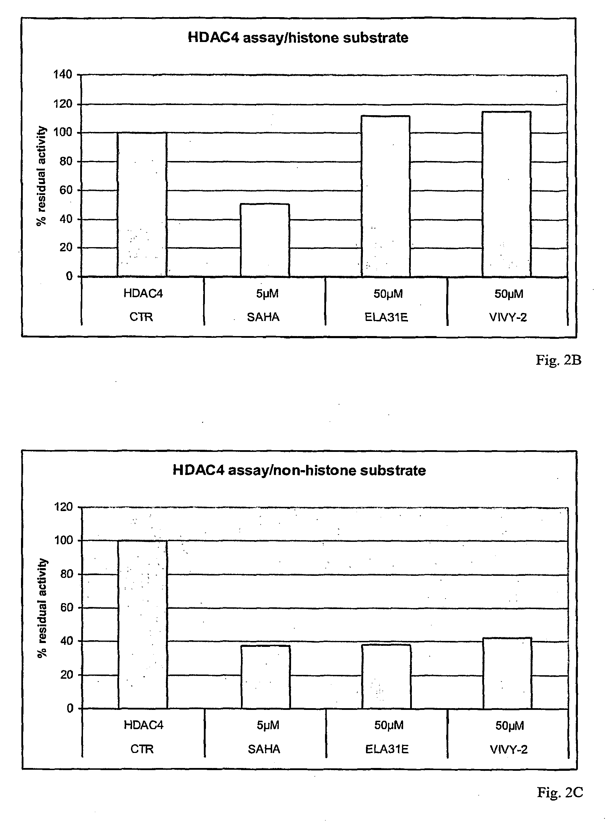

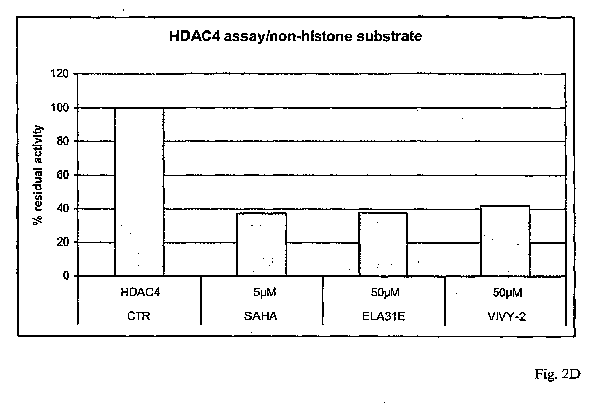

Figures 2A, 2B, 2C and 2D illustrate the HDAC biological activity of the compounds: N-(5-(4-Amino-pyridin-3-yl-carbamoyl)-penthyl]-nicotinamide hydrochloride and N-{6-[(4-aminopyridin-3-yl)-amino]-6-oxohexyl}-benzamide hydrochloride.

### Detailed description of the invention

For the purposes of the present invention, it is understood that the factors influencing the bioavailability of a drug, meaning by bioavailability the fraction of the drug absorbed in the systemic circulation, depend on a sequence of kinetic processes. These processes comprise in: 1) the disaggregation of the formulation with consequent release of the drug; 2) the dissolution of the drug in an aqueous milieu, and 3) absorption in the systemic circulation across the cell membranes. It often happens that formulations with the same active ingredient and with the same excipients, but prepared by different manufacturers, or by the same manufacturer, but belonging to different batches, present differences in bioavailability.

These difference are observed above all in the case of oral formulations of poorly soluble and slowly absorbed drugs and depend on the crystalline form (drug polymorphism), on the size of the particles and on other physical characteristics of the drug. These factors condition the dissolution of the drug, consequently influencing the rate and extent of its absorption. (Goodman & Gilman: The Pharmacological Basis of Therapeutics, 10th Ed., McGraw-Hill 2003*).*

CD/HDACI inclusion complexes according to the invention are obtained by combining, preferably in a ratio of 1:1 to 1:3, cyclodextrin (CD) and the histone deacetylase inhibitor (HDACI).

As regards the cyclodextrins, particularly advantageous for use according to the present invention is a particular class of semisynthetic cyclodextrins marketed by Roquette and indicated as HP-β-CD (hydroxypropyl-beta-cyclodextrin) and M-β-CD (methyl-beta-cyclodextrin). The first of these, marketed under the name Kleptose HP®, comes as an amorphous white powder, has an average molecular weight of 1399 daltons, and 65% and 80% solubility in water (g/100 ml) at 25°C and 50°C, respectively. It has a maximum moisture content of 5%.

The second, marketed under the name Kleptose Crysmeb®, is a mixture of cyclodextrins containing from one to seven methyl groups. Kleptose Crysmeb exp® has an average of 4 methyls per molecule of cyclodextrin.

Generally, the maximum increase in absorption is obtained when the minimum amount of cyclodextrin necessary for the solubilization of the drug is used; in fact, a substantial excess of cyclodextrin reduces the free fraction of the drug and its consequent availability.

The preparation process according to the present invention is based on the formation of inclusion complexes with cyclodextrins with the aid of microwaves. According to the invention, the process involves the following steps:
i) an aqueous solution containing cyclodextrin is prepared. Cyclodextrins in water can form saturated solutions, but for the purposes of the present invention the amount of cyclodextrin in solution will be such as to to be able to obtain a CD:HDACI ratio ranging from 1:1 to 1:5, preferably from 1:1 to 1:3, and more preferably from 1:1 to 1:2;
ii) an amount of active ingredient, consisting in the HDAC SAHA, is added to the solution such that inhibitor:cyclodextrin ratio is in the above-mentioned range. Given the poor solubility of the HDAC inhibitor, the mixing should preferably be done under shirring, for example, by means of a mechanical stirrer or mixer, so as to favour a homogeneous dispersion of the inhibitor in the aqueous solution containing cyclodextrin;
iii) the mixture thus obtained, which initially has the appearance of a dispersion, is solubilized by means of the use of microwaves. The microwaves used have a frequency typically ranging from 300 MHz to 300 GHz, and are applied at a power below 100 Watts, preferably in the range from 40 to 80 Watts, and more preferably around 50 Watts, for a time period ranging from 1 to 60 minutes. Generally, times ranging only from 2 to 30 minutes, preferably 2 to 10 minutes, make it possibile to obtain complete dissolution of the HDAC inhibitor in the aqueous cyclodextrin solution;
iv) optionally, the solution obtained in iii) is brought to dryness, for example by lyophilization.

The dried product, which is the inclusion complex according to the invention, is generally an amorphous solid which has the characteristic of being highly soluble in water.

It can be formulated in liquid or solid form with the addition of pharmacologically acceptable additives or excipients. Particularly preferred are formulations for parenteral, oral, inhalatory or topical administration.

The process according to the invention is particularly advantageous for industrial use because the inclusion product is obtained without solvents other than water and therefore does not require additional purification steps; moreover, as a lyophilized product, it can be used as is for suitable preparations of pharmaceutical formulations.

As is well known to the expert in the field, the pharmaceutical compositions according to the invention contain, in addition to the inclusion compound and related pharmaceutically acceptable salts, pharmaceutically acceptable liquid or solid carriers, solvents, emulsifying agents, sweeteners, thickening agents, buffering substances, aromatizing agents, stabilizing agents, ballast agents, binding agents, disintergrating agents, lubricating agents, vegetable oils, waxes of natural origin, cleansing agents, disinfectants.

The processes for the preparation of these compositions involve the mixing or blending of the active ingredient with the additional components.

The compositions according to the invention can be formulated in various pharmaceutical forms for oral, systemic and local use, such as, for example, tablets, capsules, granules, powders, syrups, drops, solutions for intravenous therapy, solutions for parenteral use, solutions for intramuscular and local injection therapy, solutions for enemas, ointments, creams, lotions, solutions for inhalatory therapy.

The dosages for administration of the active ingredient contained in the complex vary according to the type of disease or condition of the subjects to be treated. Inclusion products were prepared by the inventors initially without the aid of microwaves. Apart from the substantially longer preparation times - 4 to 5 days in this case, as compared to times of the order of only a few minutes with microwaves - the inventtors unexpectedly discovered, on the basis of studies conducted on lyophilized compounds using DSC (Differential Scanning Calorimetry) and solid-state NMR (Nuclear Magnetic Resonance) techniques, that these new inclusion products present structural characteristics that differ from those of inclusion products prepared without the aid of microwaves, as well as displaying greater activity *in vivo.* It can therefore be affirmed that the two preparation techniques yield inclusion complexes with different physical and pharmacological characteristics.

The inclusion products thus obtained also have the advantage that their activity is independent of the administration route and therefore do not present the variability of absorption typical of poorly soluble molecules. In fact, with the method according to the invention soluble inclusion complexes are obtained that present better wettability and an increased solubility, all of which manifests itself in the form of a better pharmacokinetic profile.

By virtue of their solubility, these inclusion complexes can be advantageously administered by the oral, parenteral and topical routes, thus avoiding first-pass metabolism (presystemic elimination that subtracts a certain amount of the drug from the general circulation at the first pass, by means of biotransformation or binding, thereby reducing the bioavailability of the drug after oral administration). The inhalatory and topical routes are particularly preferred administration routes for the purposes of the invention because they are easy to use. For use via the inhalatory route, the complexes are diluted in suitable solvents, generally aqueous, and sprayed in the form of aerosols on the mucosal surfaces of body cavities accessibile from the outside. For topical use, the complexes are mixed with suitable vehicles, obtaining emulsions that can be smeared on the surface of the skin. Another possible topical application is that which uses transdermal therapeutic systems (patches and the like) to be applied on the epidermis in such a way that the active ingredient is released and absorbed by the skin in a continuous and virtually constant manner over time.

The following examples are to be regarded as illustrating the invention.

### Examples

Hyclioxypropyl-β-cyclodextrin is a Roquette Freres product.

The deuterated solvents for the NMR analysis were purchased from Cambridge Isotope Lab.

All the other solvents and reactants used, unless otherwise specified, are of analytical grade.

The UV spectra were obtained with a UV/Vis. Perkin Elmer Lambda 40 model double-ray spectrophotomter; the software used for the data processing was the Perkin Elmer Corporation UV/WinLab software package, version 2.0.

The molar extinction coefficient values are expressed in M⁻¹ cm⁻¹.

The IR spectra were obtained with a Perkin Elmer spectrophotometer and Precisely Universal ATR Sampling Accessory.

The signals are expressed in wave numbers (cm⁻¹).

The ¹H-NMR spectra were obtained with a Bruker AMX-400 spectrophotometer.

The chemical shifts are expressed in ppm (delta) vs. tetramethylsilane (TMS) as the internal reference standard; the coupling constants are expressed in Hertz.

The solid-state NMR spectra were obtained with a Bruker Avance 600 MHz spectrophotometer in magic angle, cross-polarization conditions.

The software used for the data processing was the Bruker Daltonik GmbH WIN-NMR software package, versione 6.1.0.0.

The microwave oven used was the CEM Discover model.

### Synthesis of . 6-[(pyridin-3-carbonyl)-amino]-esanoic acid (I) and 6-[(fenilcarbonyl)-amino]-esanoic acid (II) (Reference example)

6-aminocaproic acid (1.0mmol) was suspended in dichloromethane, triethylamine (3.5mmol) and trimethylchlorosilane (1.1mmol) were then added and the suspension was refluxed under stirring for 2 hours.

The obtained suspension was then cooled for 45 min at 0°C and thereafter 1 mmol of benzoyl chloride or nicotinoyl chloride hydrochloride was added. The suspension was stirred 24 hours at room temperature and then the solvent removed. The bulky residue was washed with cold water and the white solid obtained separated by centrifugation and dried under reduced pressure.

### Synthesis of N-[5-(4-Amino-pyridin-3-yl-carbamoyl)-penthyil]_ nicotinamide (III) and N-{6-[(4-aminopyridin-3-yl)amino]-6-oxohexyl} benzamide (IV) (Reference examples)

1 mmol of acid I or II was suspended in acetonitrile, 1.3 mmol of anhydrous triethylamine and 1.2 mmol of ethylchloroformate were then added.

To the obtained suspension, kept 15 min at 0°C, were dropwise added 3.0 mmol of 3,4-diaminopyridine previously dissolved in acetonitrile.

The reaction mixture was refluxed for 4 hours and then kept overnight at room temperature. The obtained white precipitate was filtered off and the solvent removed under reduced pressure. The crude residue was purified by mean of silica gel column chromatography.

From amides III and IV were prepared the correspondent hydrochlorides (V and VI) by treatment with a saturated solution of HCl_{(g)} in diethylether.

The synthesis of compounds I, II, III, IV, V and VI was conducted according to the following scheme. 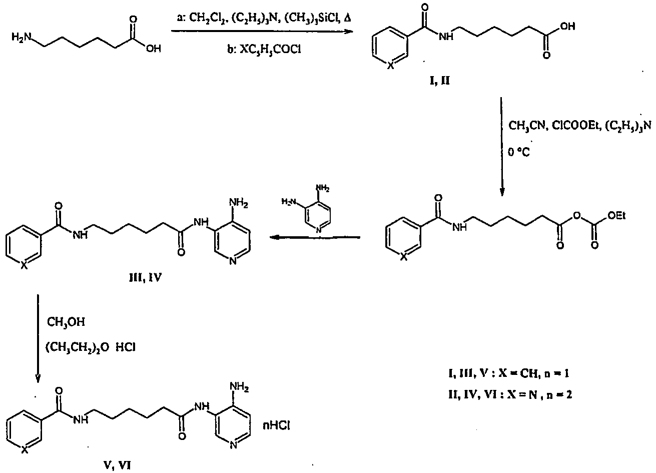
V: IR (cm⁻¹) 3334, 3196, 2924, 2853, 1681, 1661, 1502, 1304, 1139, 799.
   NMR see table 1
   mp 180°C
VI: IR (cm⁻¹) 3343, 3180, 2945, 2851, 1710, 1661, 1616, 1501, 1160, 829.
   NMR see table 1
   mp 138°-140°C

**Table 1: ¹H-NMR data of hydrochlorides V and VI expressed as □ (ppm) with respect to TMS as internal reference or to D₂O signal (4.78 ppm)**

| | | | | |
|---|---|---|---|---|
| 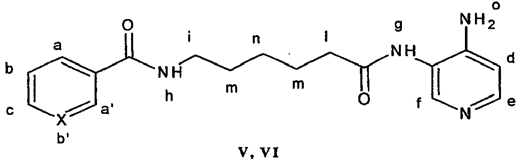 | | | | |

| | VI (DMSO) | | V (D₂O) | |
|---|---|---|---|---|
| | □ (ppm) | | □ (ppm) | |
| a | 7.81 | m 2H | 8.80 | dd 1H |
| a' | | | 9.07 | d 1H |
| b | 7.50 | m 2H | 8.03 * | m 1H |
| b' | | | | |
| c | 7.44 | m 1H | 8.72 | dt 1H |
| d | 7.94 | d 1H | 8.00 * | m 1H |
| e | 8.30 | d 1H | 8.15 ° | m 1H |
| f | 8.03 | d 1H | 8.15 ° | m 1H |
| g | 9.99 | bs 1H | exchanges | |
| h | 8.43 | bt 1H | exchanges | |
| i | 3.25 | t 2H | 3.42 | t 2H |
| l | 2.56 | t 2H | 2.62 | m 2H |
| m | 1.58 | m 4H | 1.74 | m 4H |
| n | 1.35 | m 2H | 1.45 | m 2H |
| o | 6.30 | s 2H | exchanges | |

| | | | | |
|---|---|---|---|---|
| * overlapped; ° overlaped | | | | |

### Synthesis of 4-methylnicotinamide (reference example)

The synthesis is conducted according to the following scheme.

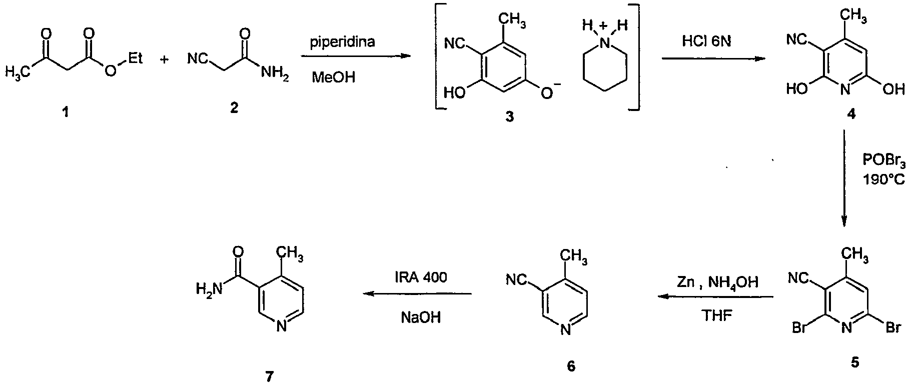

Cyanoacetamide (2) (4 g; 48 mmol) and piperdine are heated in MeOH (30 ml) at 65°C in a two-necked flask until complete dissolution; to the mixture are added 6 ml of ethyl acetoacetate (1) (6 g; 46 mmol, d₂₀ = 1.01) dropwise, reflux heating the solution for 24 hours with coolant and a CaCl₂ stopper. Subsequently leaving the reaction flask to cool, a white precipitate is formed corresponding to the piperidine salt of 2,6-dihydroxy-3-cyano-4-methylpyridine (3) which is fitered and washed with methanol.

From the salt dissolved in water, a precipitate (4) (5 g; 80 mmol, yield 90-95%) is obtained by addition of HCl 6N (a few millilitres), which is filtered, washed with water and crystallized by ethanol or methanol, and identified as 2,6-dihydroxy-3-cyano-4-methylpyridine (4) (m.p. 229-235°C).

A mixture of 2,6-dihydroxy-3-cyano-4-methylpyridine (2 g; 13.3 mmol) and phosphoryl bromide (POBr₃) (7.6 g; 26.6 mmol) is heated to melting point and then refluxed for 2 hours at 190°C in a flask immersed in a sand bath and then left to cool at room temperature. Ice is added to decompose the excess POBr₃ and, when the ice has dissolved, the aqueous suspension is extracted with three portions of CHCl₃ of 50 ml each.

From the pooled chloroform extracts, dried on anhydrous Na₂SO₄, and evaporated, 2,6-dibromo-3-cyano-4-methylpyridine (5) is obtained. The purification of (5) is obtained by chromatography on a silica gel column (Kieselgel 60), using a product:silica ratio of 1:50 (w/w) amd using CHCl₃ as the eluent phase. The only fraction that is fluxed is collected and brought to dryness, inasmuch as the various impurities are retained in the column (3.3 g; 28 mmol, yield 90%; m.p. 137-140°C).

To the 2,6-dibromo-3-cyano-4-methylpyridine (1 g; 3.6 mmol) dissolved in tetrahydrofuran (THF) (25 ml) are added 100 ml of a 30% solution of NH₄OH containing 5 g of Zn in powder form and the suspension is reflux heated for 5 hours.

At the end of this time period, the reaction mixture is left to cool to room temperature, then filtered to eliminate the excess Zn that has not reacted and extracted with three portions of CHCl₃ of 50 ml each. The chloroform extract, dried on anhydrous Na₂SO₄ and evaporated, yields 350 mg of 3-cyano-4-methylpyridine (6) which is purified by means of chromatography on a silica gel column containing 35 g of Kieselgel 60, using a product:silica ratio of 1/100 (w/w) and eluting with a mixture of CHCl₃/AcOEt 6/4 (v/v).

Collecting the first and only fluxed product in the first 400 ml of mobile phase, 3-cyano-4-methylpyridine (6) is obtained (330 mg; 3 mmol, yield 80%; m.p. 45-46°C).

4-methylnicotinamide (7) is prepared starting from the intermediate product (6) by treatment with the ion-exchange resin Amberlite IRA-410.

The resin (400 mg), preconditioned in 10% aqueous NaOH (5 ml) for 90 minutes and washed with degassed water until the excess alkalinity disappears, is added with an aqueous solution of (6) (200 mg; 1.7 mmol), reflux heating for approximately one hour.

At the end of this period the solution is filtered and the resin is washed thoroughly with 10 ml of boiling water: on adding the washing water to the filtrate, a solid residue is obtained by lyophilization, consisting in practically pure 4-methylnicotinamide (7) (220 mg; 1.6 mmol; yield 85%; m.p. 160-161°C).

### Calculation of the extinction of SAHA by UV spectrophotometry.

4.4 mg of SAHA (0.016 mmol) are weighed in a 100 ml round-bottomed flask to which are added 50 ml of methanol, brought to volume with distilled water.

An absorbance scan vs λ is done in the 200-450 nm range, allowing identification of maximum absorption at 240 nm corresponding to an absorbance of 2.1548 AU. The ε value calculated, knowing the concentration, is 12980.

At this point a series of dilutions are tested so as to obtain the most representative ε value possible.

A first 7:3 dilution was tested with an absorbance of 1.5302 AU and an ε value of 13191; a second 5:5 dilution was then done with an absorbance of 1.1472 AU and an ε value of 13821 and lastly a 3:7 dilution with an absorbance of 0.66914 AU and an ε value of 13436. The mean ε value was 13357.

To the flask containing the mother solution 2-hydroxypropyl-β-cyclodextrin is then added in increasing amounts and the spectrophotometric readings are taken, again at 240 nm.

Cyclodextrin was added in a molar ratio of 1:5, registering an absorbance of 2.1619 AU and, taking into account the changes in volume due to the dilutions, an ε value of 13263 was calculated; with the addition of cyclodextrin in a molar ratio of 1:10 the absorbnce was 2.1554 AU and the ε value was 13305; lastly, with a molar ratio of 1:15 the absorbance was 2.1833 AU and the ε value 13731.

The changes in absorbance observed following the addition of cyclodextrin prove not to be significantly different from those obtained in the absence of cyclodextrin, indicating that, in methanol, at the concentrations used for the absorbance measurements, there is complete solubilization of SAHA. Moreover, following the addition of 2-hydroxypropyl-β-cyclodextrin, no significant changes in the ε value are observed, indicating the lack of interaction with the chromophore.

### Solubility tests on SAHA by means of UV spectrophotomety,

Five aqueous solutions of SAHA in cyclodextrin were prepared, maintaining a constant amount of drug and gradually increasing the cyclodextrin concentraion.

Two mg of SAHA (0.0075 mmol) are weighed in five assay tubes and to each is added 1 ml of a solution of 2-hydroxypropyl-β-cyclodextrin (HPβCD) in double-distilled water; we started with a molar ratio of 1:1 corresponding to 0.0116 g of cyclodextrin, proceeding then with molar ratios of 1:2 corresponding to 0.0232 g, 1:3 corresponding to 0.035 g, 1:4 corresponding to 0.046, up to a final ratio of 1:5, corresponding to 0.058 g of cyclodextrin.

The tubes thus prepared and well sealed are left for one week under stirring in a mechanical stirrer at room temperature.

At the end of this period, the solutions are filtered with a 0.5 µm diameter membrane filter in five 10 ml round-bottomed flasks brought to volume with double-distilled water, and the spectrophotometric readings are taken at 240 nm.

**Table 2**

| Absorbance values measured for solutions of SAHA and HPβCD in increasing molar ratios | | |
|---|---|---|
| Molar ratio | Assorbance (AU) | Concentration (mM) |
| 1:1 | 1.6862 | 0.126 |
| 1:2 | 2,5591 | 0.191 |
| 1:3 | 2.6196 | 0.196 |
| 1:4 | 2.7752 | 0.207 |
| 1:5 | 2.8932 | 0.216 |

The data reported in Table 2 show an increase in absorbance values as a function of the increase in cyclodextrin concentration: the greatest increase was obtained on going over from the 1:1 to the 1:2 molar ratio.

From the mean value of ε calculated previously, and using Lambert and Beer's law, the millimolar concentrations of the five solutions can be calculated, as shown in the table.

An increase in solubility was therefore observed with the increase in cyclodextrin concentration, especially when going over from the 1:1 to the 1:2 solution, as reflected by an increase in absorbance.

With the concentration values thus calculated, a solubility curve for SAHA could be plotted as a function of the cyclodextrin concentration. Lastly, the solutions were lyophilized to eliminate the water and submitted to ESI-FT-ICR analysis, confirming that formation of the complexes had taken place.

On the basis of the solubility data obtained we decided to prepare the SAHA-HPβCD complexes in a molar ratio of 1:5 in that, in these conditions, maximum solubility was observed and the increases in solubility as a result of the addition of higher concentrations of HPβCD proved to be minimal.

### Preparation of the inclusion complex SAHA-HPβCD in a molar ratio of 1:5.

To 5 mg of SAHA (0.0189 mmol) are added 2 ml of an aqueous solution of cyclodextrin, dissolving 0.145 g of 2-hydroxypropyl-β-cyclodextrin (0.094 mmol) in double-distilled water.

The solution is left to stir at approximately 60°C for a few days until a perfectly clear solution is obtained; at this point the water is removed by lyophilization.

140 mg of white lyophilized product are obtained.

This product was submitted to ¹H-NMR analysis in D₂O as is and following the addition of cyclodextrin up to a molar ratio of 1:5 corresponding to 0.116 g of cyclodextrin (0.075 mmol). The respective signals are indicated in Table 3. 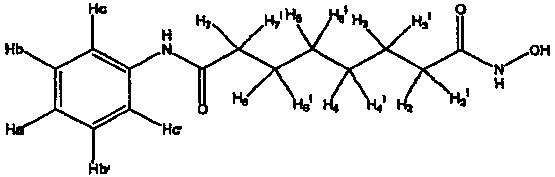 SAHA

**Table 3**

| ¹H chemical shifts in D₂O of aromatic protons of SAHA alone and of inclusion complexes with SAHA-HPβCD in a molar ratio of 1:5 prepared a) with the classic method, b) with microwaves | | | |
|---|---|---|---|
| | Hc,Hc' | Hb,Hb' | Ha |
| SAHA | 7.46 | 7.46 | 7.30 |
| SAHA-HPβCD 1:5 Classic method | 7.48 | 7.31 | 7.14 |
| SAHA-HPβCD 1:6 Microwaves | 7.57 | 7.40 | 7.22 |

### Preparation of the inclusion complex SAHA-Methyl-β-CD in a ratio of 1:2

To 5 mg of SAHA (0.0189 mmol) are added 2 ml of an aqueous solution of cyclodextrin prepared by dissolving 0.045 g. of methyl-beta-cyclodextrin (0.0378 mmol) in double-distilled water. The solution is left to stir at approximately 50°C for a few days until a perfectly clear solution is obtained; after filtration of the resulting solution, the water is removed by lyophilization. 40 mg of white lyophilized product are obtained.

¹H-NMR analysis in D₂O confirms that the complexation has taken place.

### Preparation of the inclusion complex valproic acid N-hydroxyamide:cyclodextrin in a ratio of 1:4. (reference example)

To 5 mg of valproc acid N-hydroxyamide (0.031 mmol) are added 2 ml of a solution prepared by dissolving 0.193 g of hydroxypropyl-β-cyclodextrin (0.125 mmol) in double-distilled water.

The solution is left to stir until a clear solution is obtained after which the water is removed by llyophilization.

190 mg of white lyophilized product are obtained.

The product was submitted to ¹H-NMR analysis in D₂O, as is and following the addition of cyclodextrin up to a molar ratio of 1:15, corresponding to 0.58 g of cyclodextrin (0.377 mmol). The respective signals are indicated in Table 4.

**Table 4**

| ¹H chemical shifts of valproic acid N-hydroxyamide and its inclusion complexes in HPβCD in molar ratios from 1:4 to 1:15 in D₂O. | | | | |
|---|---|---|---|---|
| | H₁ | H₂,H_{2'},H₃,H_{3'}. | H₄,H_{4'},H₅,H_{5'} | H₆,H_{6'},H_{6''},H₇,H_{7'},H,_{7''} |
| VPAI | 2.09 | 1.33 | 1.30 | 0.77 |
| VPAI-HPβCD 1:4 | 2.17 | - | - | 0.90 |
| VPAI-HPβCD 1:15 | - | - | - | 1.40 |

| | | | | |
|---|---|---|---|---|
| - signals covered by cyclodextrin | | | | |

### Preparation of inclusion complexes by means of microwaves.

One of the methods used for the preparation of inclusion complexes in cyclodextrin was the use of microwaves to favour the solubilization of the product in the aqueous solution.

The procedure is the same as that described previously (the same molar ratios described above are used), the only difference being that solubilization was obtained with microwaves at a temperature of 50°C, a pressure of 50 PSI amd a power of 50 Watts for 10 minuties; the clear solution thus obtained is in any case filtered; removal of water by lyophilization yields an amorphous white solid, which possesses the characteristic of being highly soluble in water.

The analyses carried out on the solid thus obtained by means of techniques such as DSC and on D₂O solutions by means of NMR indicate that complexation has taken place (see Table 3).

### Biological tests

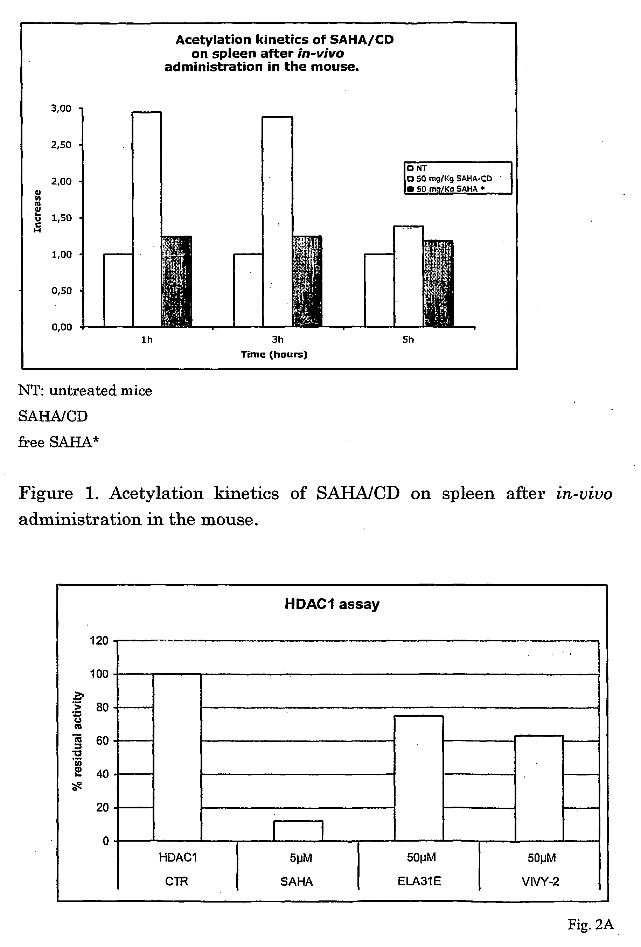

The SAHA included in cyclodextrin prepared as described above was tested both *in vitro* and *in vivo*. In particular, acetylation tests were conducted on mouse spleen, the results of which are shown in the graph in Figure 1.

The compound included in cyclodextrin was compared with free SAHA after one, three and five hours. The results obtained indicate that SAHA complexed with CD and treated with microwaves displays an activity which is roughly double that of the free compound after one and three hours, whereas after five hours the results are comparable.

In conclusion, we may affirm that the cyclodextrins, in addition to increasing the solubility of such drugs and thus their bioavailability, also surprisingly increase their activity when the complexes are prepared with the aid of microwaves.

On the basis of an initial investigation conducted on solid-state NMR spectra, we can say that the complex obtained with the aid of microwaves presents a more disorderly (amorphous) solid structure compared to the complex obtained without the aid of microwaves; moreover, also the proton resonance spectra of the two complexes generated in a D₂O solution present particularly evident substantial differences in the chemical shift values of the protons of the aromatic portion of SAHA, in agreement with the fact that it is precisely this part of the molecule that most interacts with cyclodextrin.

Fluorimetric human recombinant HDAC1 and 4 Assays. The HDAC Fluorescent Activity Assay for HDAC1 and 4 is based on the Fluor de Lys Substrate and Developer combination (BioMol) and has been carried out according to supplier's instructions and as previously reported. First, the inhibitors and purified recombinant HDAC1 or 4 enzymes have been preincubated at RT for 15 minutes in assay buffer before substrate addition, the Fluor de Lys Substrate, which comprises an acetylated lysine side chain, or alternatively the HDAC4-specific substrate reported by Lahm et al. in Proc Natl Acad Sci USA 2007, 104, 17335-17340. Briefly, for HDAC1, and HDAC4, 100 ng of recombinant proteins have been used per assay, respectively. Full length HDAC1 and 4 with C-terminal His tag were expressed using baculovirus expression systems. Deacetylation sensitizes the substrates that, in the second step, treatment with the developer produces a fluorophore. Fluorescence has been quantified with a TECAN Infinite M200 station.

VIVY-2 and ELA31E indicate N-[5-(4-Amino-pyridin-3-yl-carbamoyl)-penthyl]-nicotinamide and N-{6-[(4-aminopyridin-3-yl)aminol-6-oxohexyl} benzamide hydrochlorides respectively (which do not form part of the present invention).

## Claims

1. Process for the preparation of inclusion complexes comprising cyclodextrins and the histone deacetylase inhibitor SAHA **characterized in that** it comprises the following steps:
i) prepare an aqueous solution containing cyclodextrin;
ii) add to said solution an amount of SAHA such that the SAHA:cyclodextrin ratio is in the range 1:1 to 1:5 and under stirring so that a homogeneous dispersion of the inhibitor is formed in the aqueous solution containing cyclodextrin;
iii) subject the dispersion obtained in ii) to the application of microwaves with a frequency ranging from 300 MHz to 300 GHz, at a power below 100 Watts, so as to obtain a clear solution;
iv) optionally, submit the solution obtained in iii) to lyophilization.

2. Inclusion complexes comprising cyclodextrins and the histone deacetylase inhibitor SAHA obtainable by the process of claim 1.

3. Inclusion complexes according to claim 2, **characterized in that** said cyclodextrins are selected in the group consisting of hydroxypropyl-beta-cyclodextrins and methyl-beta-cyclodextrins.

4. Inclusion complexes according to claims 2-3, **characterized in that** the ratio of cyclodextrins to histone deacetylase inhibitors ranges from 1:1 to 1:5.

5. Inclusion complexes according to claims 2-4, **characterized in that** when the cyclodextrin is hydroxypropyl-beta-cyclodextrin and its ratio with SAHA is 1:5 and the preparation is made by using microwaves the chemical shift values 7.31 H_{b},H_{b'}, 7.48 H_{c},H_{c'}, and 7.14 Hₐ move respectively to 7.40 H_{b},H_{b'}, 7.57 H_{c},H_{c'} and 7.22 Hₐ with respect to the corresponding complex prepared in water.

6. Inclusion complexes according to claims 2-5 **characterized in that** they are used as medicaments.

7. Pharmaceutical compositions, **characterized in that** they comprise inclusion complexes according to claims 2-5 and pharmaceutically acceptable excipients and/or vehicles.

8. Pharmaceutical compositions according to claim 7, in which the excipients and/or vehicles are selected from the group consisting of: liquid or solid carriers, solvents, emulsifying agents, sweeteners, thickening agents, buffering substances, aromatizing agents, stabilizing agents, ballast agents, binding agents, disintegrating agents, lubricating agents, vegetable oils, waxes of natural origin, cleansing agents, disinfectants.

9. Pharmaceutical compositions according to claims 7-8 formulated in the form of tablets, capsules, granules, powders, syrups, drops, controlled-release skin devices, solutions for intravenous therapy, solutions for parenteral use, solutions for intramuscular and local injection therapy, solutions for enemas, ointments, creams, lotions, solutions for inhalatory therapy.

10. Use of the inclusion complexes according to claims 2-5, for the preparation of a medicament.

11. Use of the complexes according to claims 2-5 for the preparation of a medicament for the treatment of the following diseases: P. falciparum and tripanosomiases caused by leishmania and tripanosoma; diseases related to ageing, mental retardation and neurodegeneration; anxiety, osteoarthritis, psychoses, cancer, type I diabetes, epilepsy, spinal muscular atrophy.

12. Use according to claims 10-11 in which the medicament is formulated in pharmaceutical forms for oral, parenteral, inhalatory, topical, systemic and local use.

## Patentansprüche

1. Verfahren zur Herstellung von Einschlusskomplexen, umfassend Cyclodextrine und den Histondeacetylase-Inhibitor SAHA, **dadurch gekennzeichnet, dass** das Verfahren die folgenden Schritte umfasst:
(i) Herstellen einer wässrigen Lösung enthaltend Cyclodextrin;
(ii) Hinzufügen zu dieser Lösung einer Menge von SAHA, so dass das SAHA:Cyclodextrin-Verhältnis im Bereich von 1:1 bis 1:5 liegt und unter Rühren, so dass eine homogene Dispersion des Inhibitors in der wässrigen Lösung, enthaltend Cyclodextrin, gebildet wird;
(iii) Unterziehen der in (ii) erhaltenen Dispersion der Anwendung von Mikrowellen mit einer Frequenz im Bereich von 300 MHz bis 300 GHz, bei einer Leistung unter 100 Watt, um eine klare Lösung zu erhalten;
(iv) gegebenenfalls Unterziehen der in (iii) erhaltenen Lösung einer Lyophilisierung.

2. Einschlusskomplexe, umfassend Cyclodextrine und den durch das Verfahren gemäß Anspruch 1 erhaltbaren Histondeacetylase-Inhibitor SAHA.

3. Einschlusskomplexe nach Anspruch 2, **dadurch gekennzeichnet, dass** die Cyclodextrine ausgewählt sind aus der Gruppe, bestehend aus Hydroxypropyl-beta-cyclodextrinen und Methyl-beta-cyclodextrinen.

4. Einschlusskomplexe nach den Ansprüchen 2-3, **dadurch gekennzeichnet, dass** das Verhältnis von Cyclodextrinen zu Histondeacetylase-Inhibitoren im Bereich von 1:1 bis 1:5 liegt.

5. Einschlusskomplexe nach den Ansprüchen 2-4, **dadurch gekennzeichnet, dass**, wenn das Cyclodextrin Hydroxypropyl-beta-cyclodextrin ist und sein Verhältnis mit SAHA 1:5 ist und die Herstellung unter Verwendung von Mikrowellen durchgeführt wird, die chemischen Verschiebungswerte 7,31 H_{b},H_{b'}, 7,48 H_{c},H_{c'} und 7,14 Hₐ jeweils zu 7,40 H_{b},H_{b'}, 7,57 H_{c},H_{c'} bzw. 7,22 Hₐ verschoben sind in Bezug auf den entsprechenden in Wasser hergestellten Komplex.

6. Einschlusskomplexe nach den Ansprüchen 2-5, **dadurch gekennzeichnet, dass** sie als Arzneimittel verwendet werden.

7. Pharmazeutische Zusammensetzungen, **dadurch gekennzeichnet, dass** sie Einschlusskomplexe nach den Ansprüchen 2-5 und pharmazeutisch verträgliche Hilfsstoffe und/oder Vehikel umfassen.

8. Pharmazeutische Zusammensetzungen nach Anspruch 7, in der die Hilfsstoffe und/oder Vehikel ausgewählt sind aus der Gruppe, bestehend aus: flüssigen oder festen Trägerstoffen, Lösemitteln, Emulgatoren, Süßungsmitteln, Verdickungsmitteln, Pufferstoffen, aromatisierenden Mitteln, Stabilisierungsmitteln, Ballastmitteln, Bindemitteln, Aufschlussmitteln, Schmiermitteln, pflanzlichen Ölen, Wachsen natürlichen Ursprungs, Reinigungsmitteln, Desinfektionsmitteln.

9. Pharmazeutische Zusammensetzungen nach den Ansprüchen 7-8 in der Form von Tabletten, Kapseln, Granulaten, Pulvern, Sirupen, Tröpfchen, Vorrichtungen zur kontrollierten Freisetzung auf der Haut, Lösungen zur intravenösen Therapie, Lösungen zur parenteralen Verwendung, Lösungen zur intramuskulären und lokalen Injektionstherapie, Lösungen für Einläufe, Salben, Cremes, Lotionen, Lösungen zur inhalatorischen Therapie formuliert.

10. Verwendung der Einschlusskomplexe nach den Ansprüchen 2-5 zur Herstellung eines Arzneimittels.

11. Verwendung der Komplexe nach den Ansprüchen 2-5 zur Herstellung eines Arzneimittels zur Behandlung der folgenden Krankheiten: P. falciparum und Trypanosomiasis, verursacht durch Leishmania und Trypanosoma; Krankheiten, die mit dem Altern, geistiger Retardierung und Neurodegeneration in Verbindung stehen; Angst, Osteoarthritis, Psychosen, Krebs, Typ I-Diabetes, Epilepsie, spinaler Muskelatrophie.

12. Verwendung nach den Ansprüchen 10-11, in der das Arzneimittel in pharmazeutischen Formen zur oralen, parenteralen, inhalatorischen, topischen, systemischen und lokalen Verwendung formuliert ist.

## Revendications

1. Procédé pour la préparation de complexes d'inclusion comprenant des cyclodextrines et l'inhibiteur de l'histone déacétylase SAHA **caractérisé en ce qu'**il comprend les étapes suivantes :
i) Préparer une solution aqueuse contenant une cyclodextrine ;
ii) ajouter à ladite solution une quantité de SAHA telle que le ratio SAHA : cyclodextrine se situe entre 1 : 1 et 1 : 5 et sous agitation pour qu'une dispersion homogène de l'inhibiteur se forme dans la solution aqueuse contenant la cyclodextrine ;
iii) exposer la dispersion obtenue en ii) à des rayonnements micro-ondes d'une fréquence allant de 300 MHz à 300 GHz, d'une puissance inférieure à 100 Watts, de manière à obtenir une solution claire ;
iv) optionnellement, soumettre la solution obtenue en iii) à une lyophilisation.

2. Complexes d'inclusion comprenant des cyclodextrines et l'inhibiteur de l'histone déacétylase SAHA pouvant être obtenus par le procédé de la revendication 1.

3. Complexes d'inclusion selon la revendication 2, **caractérisés en ce que** lesdites cyclodextrines sont choisies dans le groupe formé par les hydroxypropyl-béta-cyclodextrines et les méthyl-béta-cyclodextrines.

4. Complexes d'inclusion selon les revendications 2-3, **caractérisés en ce que** le ratio cyclodextrines : inhibiteurs de l'histone déacétylase se situe entre 1:1 et 1:5.

5. Complexes d'inclusion selon les revendications 2-4, **caractérisés en ce que** quand la cyclodextrine est une hydroxypropyl-béta-cyclodextrine et son ratio avec SAHA est de 1: 5 et la préparation est faite à l'aide de micro-ondes les valeurs de déplacements chimiques 7.31 H_{b},H_{b'}, 7.48 H_{c},H_{c'} et 7.14 Hₐ sont déplacées respectivement à 7.40 H_{b},H_{b'}, 7.57 H_{c},H_{c'} et 7.22 Hₐ par rapport au complexe correspondant préparé dans l'eau.

6. Complexes d'inclusion selon les revendications 2-5, **caractérisés en ce qu'**ils sont utilisés comme médicaments.

7. Compositions pharmaceutiques, **caractérisées en ce qu'**elles comprennent des complexes d'inclusion selon les revendications 2-5 et des excipients et/ou véhicules pharmaceutiquement acceptables.

8. Compositions pharmaceutiques selon la revendication 7, dans lesquelles les excipients et/ou véhicules sont choisis dans le groupe formé par les supports solide ou liquide, les solvants, les agents émulsifiants, les édulcorants, les agents épaississants, les substances tampons, les agents aromatisants, les agents stabilisants, les agents de ballast, les agents liants, les agents de désintégration, les agents lubrifiants, les huiles végétales, les cires d'origine naturelle, les agents nettoyants, les désinfectants.

9. Compositions pharmaceutiques selon les revendications 7-8, formulées sous la forme de comprimés, de capsules, de granules, de poudres, de sirops, de gouttes, de dispositifs cutanés à libération contrôlée, de solutions pour traitement intraveineux, de solutions à usage parentéral, de solutions pour traitement par injection intramusculaire et locale, de solutions de lavement, de pommades, de crèmes, de lotions, de solutions pour traitement par inhalation.

10. Utilisation des complexes d'inclusion selon les revendications 2-5, pour la préparation d'un médicament.

11. Utilisation des complexes selon les revendications 2-5, pour la préparation d'un médicament pour le traitement des maladies suivantes : P. falciparum et les trypanosomiases causées par le leishmania et le trypanosome; les maladies liées à l'âge, à la déficience mentale et à la neurodégénérescence ; l'anxiété, l'arthrose, les psychoses, le cancer, le diabète de type 1, l'épilepsie, l'atrophie musculaire spinale.

12. Utilisation selon les revendications 10-11 dans laquelle le médicament est formulé sous forme pharmaceutique pour usage oral, parentéral, par inhalation, topique, systémique et local.
